# EUROPÄISCHE PATENTSCHRIFT

(11) **EP 2 954 926 B1**
(45) Veröffentlichungstag und Bekanntmachung des Hinweises auf die Patenterteilung: **07.08.2019**
(21) Anmeldenummer: 15171689.1
(22) Anmeldetag: 11.06.2015
(51) Int. Cl.: A61M 37/00, A01K 11/00

(54) **HANDGERÄT ZUM WIEDERHOLTEN AUFSTECHEN EINER MENSCHLICHEN ODER EINER TIERISCHEN HAUT**
HANDHELD DEVICE FOR THE REPEATED PUNCTURING OF HUMAN OR ANIMAL SKIN
APPAREIL PORTATIF POUR LA PIQÛRE RÉPÉTÉE D'UNE PEAU HUMAINE OU ANIMALE

(30) Priorität: 12.06.2014 EP 14172103
(43) Veröffentlichungstag der Anmeldung: 16.12.2015
(73) Patentinhaber: MT.DERM GmbH, 14167 Berlin (DE)
(72) Erfinder: SCHERKOWSKI, Dirk, 12489 Berlin (DE)
(74) Vertreter: Bittner, Thomas L.

(56) Entgegenhaltungen:
- CN-Y- 201 426 920
- DE-U1-202006 013 148
- GB-A- 2 044 879
- US-A1- 2012 123 462

## Beschreibung

Die Erfindung betrifft ein Handgerät zum wiederholten Aufstechen einer menschlichen oder einer tierischen Haut.

### Hintergrund

Vorrichtungen zum lokalen Aufstechen einer menschlichen oder einer tierischen Haut werden in der Regel als Handgeräte ausgeführt. Vom Bedienpersonal können derartige Handgeräte zum Aufbringen einer Farbe für eine Tätowierung (Tattoo) und / oder permanentes Make-up im Bereich der Hautoberfläche verwendet werden. Aber auch das Einbringen kosmetischer oder medizinischer Wirkstoffe über die Haut ist mit solchen Geräten möglich, indem die Haut lokal aufgestochen wird. Darüber hinaus können solche Geräte verwendet werden, ohne dass irgendeine Substanz eingebracht wird, zum Beispiel zur Hautstimulierung.

Ein Handgerät zum lokalen Aufstechen einer Haut ist beispielsweise aus der Druckschrift DE 299 19 199 U1 bekannt. Das bekannte Handgerät weist ein Griffstück, eine Antriebseinrichtung und eine Stechnadel auf, die mit Hilfe der Antriebseinrichtung im Betrieb relativ zu einer Nadeldüse vor und zurück bewegt wird, wobei mindestens zwei lösbar miteinander verbundene Module vorgesehen sind, und das eine der beiden Module als wieder verwendbares Grundmodul mit integrierter Antriebseinrichtung ausgebildet ist. Das andere der beiden Module ist ein sterilisiertes Einwegmodul, in das bei dem bekannten Handgerät alle durch die Köperflüssigkeiten eines Kunden infizierbaren Bestandteile integriert sind. Auf diese Weise wird das Handgerät in Form von zwei Modulen zur Verfügung gestellt, von denen eines, nämlich das Einwegmodul, nach der Benutzung ausgetauscht werden kann, während das andere Modul, welches die Antriebseinrichtung umfasst, wieder verwendet wird. Mit Hilfe des Einwegmoduls werden die hygienischen Bedingungen beim Aufbringen einer Tätowierung und / oder von permanentem Make-up verbessert, da alle Teile ausgetauscht werden, die potentiell durch die bei der Behandlung austretende Körperflüssigkeit des Kunden kontaminiert werden können. Es wird so vermieden, dass das gesamte Handgerät ausgetauscht werden muss.

Aus dem Dokument EP 1 495 782 A1 ist ein Antriebsmodul für eine Vorrichtung zum lokalen Aufstechen einer menschlichen oder einer tierischen Haut bekannt, bei dem eine Antriebseinrichtung, mit der eine Antriebsbewegung erzeugbar ist, und ein an die Antriebseinrichtung gekoppelter Wandlungsmechanismus vorgesehen sind, mit dem die Antriebsdrehbewegung in einer auf eine die Haut lokal aufstechende Stecheinrichtung einkoppelbare Vorwärts- / Rückwärtsbewegung umgewandelt wird, so dass eine repetierende Bewegung einer Stechnadel ermöglicht ist. Der Wandlungsmechanismus umfasst ein Funktionsbauteil, welches bei der Bewegungswandlung eine Taumel- oder Kippbewegung ausführt, wodurch eine Antriebskraft zum Bewegen einer die Haut lokal aufstechenden Nadel in Vorwärts- und Rückwärtsrichtung zur Verfügung gestellt wird. Das Funktionsbauteil ist in einer Ausführung mittels eines Kugellagers freilaufend gelagert. Ein nicht beabsichtigtes Verdrehen des Funktionsbauteils, welches durch die Antriebsdrehbewegung hervorgerufen werden kann, wird bei der bekannten Vorrichtung dadurch verhindert, dass das mittels Kugellager montierte Funktionsbauteil oder ein hieran gebildeter Vorsprung in eine Ausnehmung eingreifen. Es hat sich gezeigt, dass diese Art der Sicherung des Funktionsbauteils beim Betrieb der Vorrichtung zu einer nicht unerheblichen Geräuschbelastung führt, die insbesondere durch das Hin- und Herbewegen des Vorsprungs in der Ausnehmung verursacht wird.

Aus dem Dokument DE 20 2006 013 148 U1 ist eine Vorrichtung zum Übertragen von axial gerichteten Kräften auf Nadeln einer Tätowier- oder Schminkmaschine bekannt. Die Vorrichtung weist einen Pleuel auf, welcher eine axial gerichtete Kraft auf mindestens eine Nadel überträgt. Es sind Mittel zum Umwandeln eines Rotationsmoments in die axial gerichtete Kraft vorgesehen, wobei die Mittel zum Umwandeln so ausgestaltet sind, dass eine Variation eines auf die Nadel axial wirkenden Hubs bewirkt werden kann. Zum Übertragen der axial gerichteten Kraft auf das Pleuel dient eine Taumelscheibe, die über ein Kugellager an einer Unwuchtbuchse aufgenommen ist.

Im Dokument GB 2 044 879 A ist ein Antriebsmechanismus zum Umwandeln einer Drehbewegung in eine Vor- und Zurückbewegung offenbart. Ein Stößel, an dem eine Nadel aufgenommen ist, koppelt über ein Pleuel an einem Kurbeltrieb.

Das Dokument US 2012/0123462 A1 betrifft eine Tätowiervorrichtung. Ein Wandlungsmechanismus zum Umwandeln einer rotierenden Antriebsbewegung in eine Vor- und Zurückbewegung für die Stechnadel umfasst eine zur Rotationsachse schräg gestellte Scheibe, an welcher die Antriebsbewegung abgegriffen wird.

### Zusammenfassung

Aufgabe der Erfindung ist es, ein Handgerät zum wiederholten lokalen Aufstechen einer menschlichen oder einer tierischen Haut anzugeben, bei denen im Betrieb des Handgerätes der Bedienkomfort verbessert ist, insbesondere hinsichtlich störender Begleiterscheinungen wie zum Beispiel Geräusche oder Vibrationen des Handgerätes.

Zur Lösung der Aufgabe ist ein Handgerät zum wiederholten lokalen Aufstechen einer menschlichen oder tierischen Haut nach dem unabhängigen Anspruch 1 geschaffen. Vorteilhafte Ausgestaltungen sind Gegenstand von abhängigen Unteransprüchen.

Es ist ein Handgerät zum wiederholten lokalen Aufstechen einer menschlichen oder tierischen Haut geschaffen, welches ein Gehäuse mit einem hieran gebildeten Handgriff aufweist. Im Betrieb des Handgerätes, welches im Fall der Nutzung zum Ausbilden von Tattoos oder permanentem Makeup auch als Tätowiervorrichtung bezeichnet werden kann, greift der Nutzer den Handgriff, um das Handgerät dann zu führen. Andere Anwendungen sehen das lokale Aufstechen der Haut zum Einbringen eines kosmetischen oder medizinischen Wirkstoffes vor. Auch eine Injektion von Wirkstoffen durch Kanülen kann erfolgen. Aber auch für das lokale Aufstechen der menschlichen oder tierischen Haut ohne Einbringen irgendeiner Substanz kann das Handgerät genutzt werden, zum Beispiel zur Hautstimulierung. Im Gehäuse ist eine Antriebseinrichtung aufgenommen, mit der über eine Antriebswelle eine rotierende Antriebskraft bereitgestellt wird. Hierfür kann ein Elektromotor vorgesehen sein.

An die Antriebswelle koppelt ein Wandlungsmechanismus, der im Gehäuse angeordnet und eingerichtet ist, die rotierende Bewegung (rotierende Antriebskraft) um eine Drehachse in eine Antriebsbewegung entlang einer Antriebsbewegungsrichtung zu wandeln. Die hierdurch bereitgestellte Antriebskraft ist in einem Ausführungsbeispiel eine axial ausgerichtete Antriebsbewegung.

Es ist weiterhin eine Stecheinrichtung vorgesehen, die im Gehäuse aufgenommen ist und eine oder mehrere Stechnadeln aufweist, die an einer Nadelaufnahme angeordnet sind. Im Fall von mehreren Stechnadeln können diese in Gruppen angeordnet sein, in denen die Nadeln einander nahestehen. Aber auch die Verteilung von Nadeln oder Nadelgruppen über die Fläche einer Nadelplatte kann vorgesehen sein. In diesem Fall ist die Nadelaufnahme mit einer Nadelplatte gebildet. Die Nadelaufnahme ist verbunden mit dem Wandlungsmechanismus und wird zusammen mit der einen oder den mehreren Stechnadeln im Betrieb entlang einer Bewegungsbahn wiederholt vor und zurück bewegt, beispielsweise einer geradlinigen Bewegungsbahn, zum Beispiel in einer axialer Richtung.

Der Wandlungsmechanismus weist eine Stabkurbeleinrichtung auf, die an die Antriebswelle koppelt. Auf diese Weise kann ein Stabkurbeltrieb realisiert werden. Ein von der Stechnadel beim Vor- und Zurückbewegen ausgeführter Hub ist einstellbar, indem eine Relativlage zwischen einer Axialrichtung der Drehachse und der Antriebsbewegungsrichtung verändert wird.

Die Antriebsachse (Antriebsseite) ist nicht parallel zur Richtung der Bewegung (Abtriebsseite) angeordnet sein, zum Beispiel einer axial ausgerichteten Bewegung. Hierdurch ist eine Schrägstellung der beiden Achsen zueinander ausgebildet. Der Winkel zwischen Richtung der Antriebsachse (axiale Richtung der Antriebswelle) und Richtung der Bewegung, welcher von Null Grad verschieden ist, kann einstellbar sein. Mittels Verstellen des Winkels (Änderung der Schrägstellung) kann die Hubeinstellung ausführbar sein. Zum Verstellen des Winkels kann in einem Ausführungsbeispiel vorgesehen sein, gelenking miteinander verbundene Gehäuseteile relativ zueinander zu verlagern, sei es manuell oder mittels eines Antriebs. In einer Ausführung ist der Winkel zwischen Richtung der Antriebsachse und Richtung der Bewegung verschieden von 90 Grad.

Das Einstellen des Hubs kann manuell oder mittels eines Einstellantriebs erfolgen.

Bei dem Handgerät kann die Antriebseinrichtung, insbesondere ein Elektromotor, in einem zum Handgriff am Gehäuse querstehenden Gehäuseabschnitt aufgenommen sein. Die Antriebseinrichtung kann eingerichtet sein, im Betrieb eine drehende Antriebsbewegung von wenigstens etwa 1.800 min⁻¹ (30 Hz) bereitzustellen.

Bei einer beispielhaften Ausführung der Stabkurbeleinrichtung kann in einer Ausführung ein antriebsseitiges Kopplungselement vorgesehen sein, welches an die Antriebswelle koppelt und im Betrieb der rotierenden Antriebsbewegung / Antriebswelle entsprechend rotiert. Das antriebsseitige Kopplungselement kann in einer Ausgestaltung als Antriebsscheibe ausgeführt sein. An das antriebsseitige Kopplungselement kann ein proximales Pleuelende eines Stabpleuels der Stabkurbeleinrichtung gelenkig koppeln, derart, dass das proximale Pleuelende beim Rotieren des antriebsseitigen Kopplungselementes auf einer geschlossenen Bewegungsbahn, insbesondere Kreisbahn, um die Rotationsachse der Antriebswelle herumbewegt wird. Das antriebsseitige Kopplungselement kann direkt auf der Antriebswelle sitzen, so dass es bei deren Rotation mitbewegt wird. Hierbei läuft dann das proximale Pleuelende auf seiner geschlossenen Bewegungsbahn um die Richtung der Rotationsachse der Antriebswelle herum. Ein distales Pleuelende des Stabpleuels kann an ein abtriebseitiges Kopplungselement gelenkig koppeln, welches zum Übertragen der axial ausgerichteten Antriebsbewegung dann an den Nadelschaft koppelt, so dass im Betrieb der Nadelschaft der axial ausgerichteten Antriebsbewegung vor- und zurückbewegt wird.

Die Gelenkverbindung zwischen dem proximalen Pleuelende und dem antriebseitigen Kopplungselement und / oder die Gelenkverbindung zwischen dem distalen Pleuelende und dem abtriebseitigen Kopplungselement kann eingerichtet sein, eine räumliche Schwenkbarkeit des jeweiligen Pleuelendes gegenüber dem Kopplungselement zulassend ausgeführt sein.

Das Gehäuse kann aus einem einzigen Material oder einer Kombination verschiedener Materialien bestehen, wozu insbesondere Metall und Kunststoff gehören. Gehäuseabschnitte können lösbar montiert sein, um zum Beispiel Bereiche des Gehäuseinneren zu Austausch- oder Wartungszwecken freizugeben. Aus dem Gehäuse kann eine Kabelverbindung herausgeführt sein, die zum Anschluss des Handgerätes an ein externes Steuergerät dient. Die Kabelverbindung kann dem Anschließen eines Elektromotors an eine Energiequelle dienen, insbesondere über das Steuergerät. Die Kabelverbindung kann Datenkabel aufweisen, über die zwischen dem Handgerät und dem Steuergerät elektronische Daten austauschbar sind. Ein solcher Datenaustausch zwischen Handgerät und Steuergerät kann alternativ oder ergänzend auch über eine drahtlose Datenverbindung ausgeführt werden, zum Beispiel unter Verwendung der Bluetooth-Technologie. Steuergeräte für Handgeräte zum wiederholten lokalen Aufstechen einer menschlichen oder tierischen Haut sind als solche in verschiedenen Ausführungsformen bekannt und werden hier daher nicht näher erläutert.

In den verschiedenen Ausführungsformen kann die Kopplung zwischen Wandlungsmechanismus und Nadelaufhahme, die mit einem Nadelschaft gebildet sein kann, derart ausgeführt sein, dass im Betrieb die Nadelaufnahme aufgrund der bereitgestellten Antriebskraft nicht nur vorgefahren wird, sondern auch mittels des das Kopplungsbauteil umfassenden Wandlungsmechanismus zurückgezogen wird. Bei dieser Ausführungsform stellt der Wandlungsmechanismus selbst eine Rückstellkraft bereit. Alternativ oder ergänzend kann vorgesehen sein, dass die Rückstellkraft wenigstens teilweise von einem elastischen Element bereitgestellt wird, beispielsweise einer Feder oder einer Membran. Bei dieser Ausführung erfolgt das Aus- oder Vorfahren der Nadelaufnahme mit der einen oder den mehreren Stechnadeln gegen eine elastische Vorspannung, die ihrerseits dann zum Zurückbewegen beiträgt oder dieses allein bewirkt. Das elastische Element zieht sich nach dem Strecken beim Ausfahren der Stechnadeln selbsttätig wieder zusammen und bewirkt so die Rückbewegung der einen oder der mehreren Nadeln.

Der Wandlungsmechanismus kann eine Drehentkopplung aufweisen. Die Drehentkopplung sorgt dafür, dass ausgehend von der rotierenden Bewegung des antriebseitigen Kopplungselementes kein Drehmoment auf den Nadelschaft mit der einen oder mehreren hierin aufgenommenen Nadeln übertragen wird. Das Entkoppeln von der drehenden Antriebsbewegung kann in einer mechanischen Kette des Wandlungsmechanismus an einem oder mehreren Übergängen zwischen benachbarten Elementen der Übertragungskette vorgesehen sein.

Die gelenkige Kopplung zwischen dem proximalen Pleuelende und dem antriebseitigen Kopplungselement kann drehentkoppelnd ausgeführt sein. Die Drehentkopplung kann mit Hilfe eines Kugelkopfgelenkes ausgeführt sein, bei dem ein Kugelkopf in einer Kugelpfanne drehbar aufgenommen ist. In Verbindung mit dem proximalen Pleuelende geht es hierbei insbesondere um eine Drehung um die Längsachse des Stabpleuels. Der Kugelkopf kann an dem proximalen Pleuelende oder am antriebseitigen Kopplungselement vorgesehen sein. Anstelle der Aufnahme eines Kugelkopfes in der Kugelpfanne kann der Kugelkopf mit einer hierauf sitzenden Kugelschale versehen sein, welche ihrerseits in der Kugelpfanne drehbar aufgenommen ist, wohingegen die Kugelschale fest auf dem Kugelkopf gespannt ist.

Eine Ausführungsform sieht vor, dass die gelenkige Kopplung zwischen dem distalen Pleuelende und dem abtriebseitigen Kopplungselement drehentkoppelnd ausgeführt ist. Es gelten die obigen Ausführungen in Verbindung mit der gelenkigen Aufnahme des proximalen Pleuelendes entsprechend.

Bevorzugt sieht eine Fortbildung vor, dass das proximale Pleuelende und das distale Pleuelende am Stabpleuel drehentkoppelt aufgenommen sind. Bei dieser Ausführungsform kann sich eines der Pleuelenden unabhängig von dem anderen Pleuelende um die Längsachse des Stabpleuels drehen. In einer Ausführungsform sind proximales und distales Pleuelende mit einem sich zur Mitte des Stabpleuels hin erstreckenden Abschnitt drehbar in einer Pleuelhülse aufgenommen.

Eine Ausgestaltung kann vorsehen, dass das proximale Pleuelende mit dem antriebseitigen Kopplungselement und / oder das distale Pleuelende mit dem abtriebseitigen Kopplungselement mittels eines Kugelgelenkes gelenkig gekoppelt sind.

Eine Weiterbildung kann vorsehen, dass das abtriebseitige Kopplungselement über eine starre Verbindung mit dem Nadelschaft verbunden oder einstückig hiermit ausgeführt ist. Das abtriebseitige Kopplungselement kann mit dem Nadelschaft in einer gemeinsamen Führung im Gehäuse angeordnet sein. Alternativ können für abtriebseitiges Kopplungselement und Nadelschaft voneinander getrennte Führungen im Gehäuse vorgesehen sein. Die eine oder die mehreren Führungen können parallel oder schräg zur Rotationsachse der Antriebswelle gebildet sein. Im Fall einer einstückigen Ausführung kann die gelenkige Ankopplung des distalen Pleuelendes auf einer der Antriebseinrichtung zugewandten Stirnseite des Nadelschaftes angeordnet sein. Die Verbindung zwischen abtriebseitigen Kopplungselement und Nadelschaft kann lösbar oder nicht lösbar ausgeführt sein. Es kann eine mechanische und / oder magnetische Kopplung zum Verbindung genutzt werden.

Eine Weiterbildung sieht vor, dass an den Nadelschaft eine Rückholeinrichtung koppelt. Die Rückholeinrichtung kann zum Beispiel ein Federelement aufweisen. Mit Hilfe der Rückholeinrichtung kann eine Rückstellkraft für den Nadelschaft und / oder das abtriebseitige Kopplungselement bereitgestellt sein.

Eine Ausgestaltung sieht vor, dass ein maximaler Nadelüberstand einer Stechnadelspitze der Stechnadel in Bezug auf eine vordere Gehäuseöffnung einstellbar ist.

Das Gehäuse kann mit mehreren Gehäusemodulen gebildet sein, wobei in einem Antriebsmodul die Antriebseinrichtung und in einem Stechmodul die Stecheinrichtung angeordnet ist. Die Gehäusemodule können lösbar miteinander verbunden sein. In einer Ausgestaltung von lösbar miteinander verbundenen Gehäusemodulen kann das Stechmodul, welches auch als Nadelmodul bezeichnet werden kann, als sterilisiertes Einwegmodul ausgeführt sein. Auch ein Gehäuseteil oder -modul mit einem Handgriff kann als lösbares Modul gebildet sein. Das Gehäuseteil kann neben dem Stech- oder Nadelmodul, welches an dem Gehäuseteil lösbar angeordnet sein kann, als weiteres Einwegmodul oder als wiederverwendbares Modul ausgeführt sein. Es ist weiterhin denkbar, Stechmodul und Gehäuseteil mit Handgriff als eine Einheit auszubilden, zum Beispiel auch einstückig.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung eines Handgeräts zum wiederholten lokalen Aufstechen einer menschlichen oder tierischen Haut von der Seite,
- Fig. 2A bis 2D: eine schematische Darstellung des Handgeräts im Schnitt in verschiedenen Arbeitsstellungen, wobei ein Arbeitshub und ein Nadelüberstand maximal eingestellt sind,
- Fig. 3A bis 3D: schematische Darstellungen eines Handgeräts in verschiedenen Arbeitsstellungen im Schnitt, wobei der Arbeitshub maximal und der Nadelüberstand minimal eingestellt sind,
- Fig. 4A bis 4D: schematische Darstellungen eines Arbeitsgeräts in verschiedenen Arbeitsstellungen im Schnitt, wobei der Arbeitshub minimal und der Nadelüberstand maximal eingestellt sind,
- Fig. 5A bis 5D: schematische Darstellungen eines Arbeitsgerätes in verschiedenen Arbeitsstellungen im Schnitt, wobei der Arbeitshub und der Nadelüberstand minimal eingestellt sind,
- Fig. 6: eine vergrößerte perspektivische Darstellung eines Kopplungsbereiches,
- Fig. 7: eine vergrößerte Schnittdarstellung eines Teils des Handgeräts,
- Fig. 8A bis 8D: schematische Darstellungen einer Anordnung von Komponenten, die nicht Teil der beanspruchten Erfindung ist, für ein Handgerät in verschiedenen Arbeitsstellungen, wobei eine geradlinige Bewegungsbahn beim Vor- und Zurückfahren des Nadelschaftes und die Antriebsachse parallel versetzt zueinander angeordnet sind.

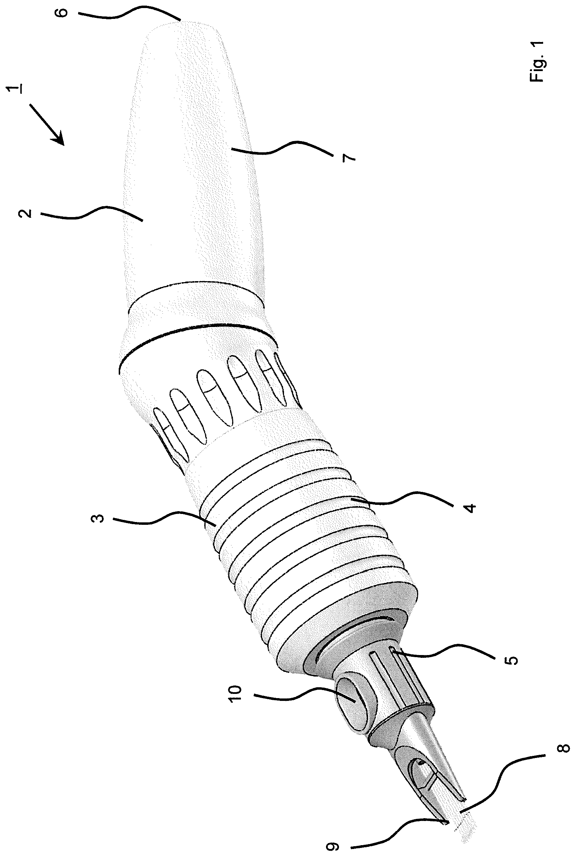

Fig. 1 zeigt eine schematische Darstellung eines Handgerätes 1 zum wiederholten lokalen Aufstechen einer menschlichen oder tierischen Haut. An einem Gehäuse 2 ist an einem vorderen Gehäuseteil 3 außen ein Handgriff oder -stück 4 gebildet. Das vordere Gehäuseteil 3 nimmt ein Stech- oder Nadelmodul 5 lösbar auf, welches als Einwegmodul ausführbar und am vorderen Gehäuseteil 3 gesichert ist, zum Beispiel mittels eines Bajonettverschlusses.

Bei der dargestellten Ausführungsform ist in dem Gehäuse 2 ein Elektromotor als Antrieb aufgenommen, welcher über eine Steckereinrichtung 6 an eine Energieversorgung angeschlossen werden kann. Es kann vorgesehen sein, dass über die Steckereinrichtung 6 auch Signal- oder Steueranschlüsse realisiert werden, zum Beispiel zum Verbinden des Handgerätes mit einer externen Steuereinrichtung (nicht dargestellt), über die zum Beispiel eine Drehzahlregelung für den Elektromotor bereitgestellt sein kann.

Das Gehäuse 2 weist ein hinteres Gehäuseteil 7 auf, welches als ein Antriebsmodul mit dem Elektromotor ausgeführt sein kann. Das vordere und das hintere Gehäuseteil 3, 7 können lösbar miteinander verbunden sein.

Im Stech- oder Nadelmodul 5 sind in üblicher Weise ein oder mehrere Nadeln 8 aufgenommen, die im Betrieb durch eine vordere Gehäuseöffhung 9 aus- und eingefahren werden können. Die eine oder die mehreren Nadeln 8 ihrerseits sind im vorderen Gehäuseteil 3 an einer Nadelaufhahme (nicht dargestellt) in üblicher Weise angeordnet.

Am Stech- oder Nadelmodul 5 ist eine Einführöffnung 10 angeordnet, über welche in einer möglichen Betriebsart ein Farbstoff einführbar ist.

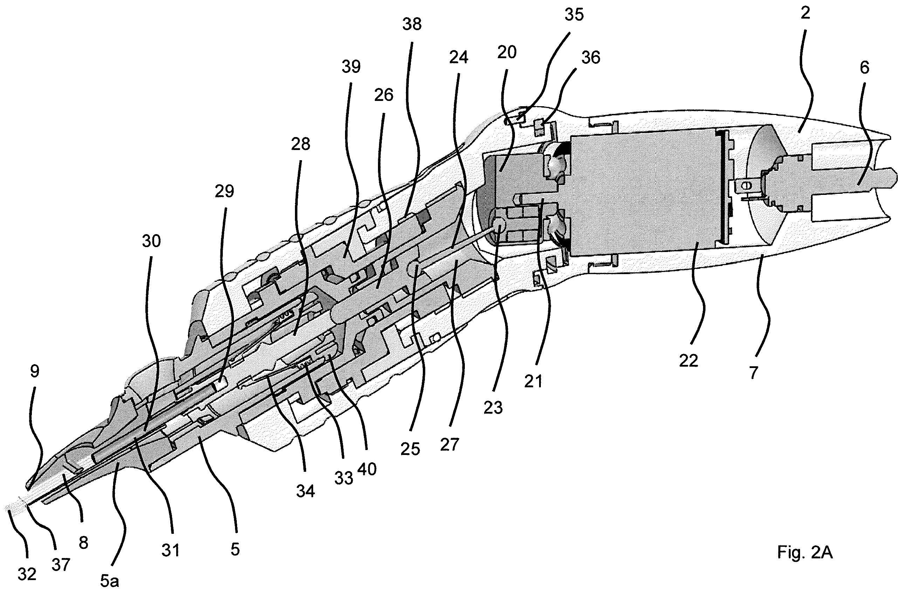

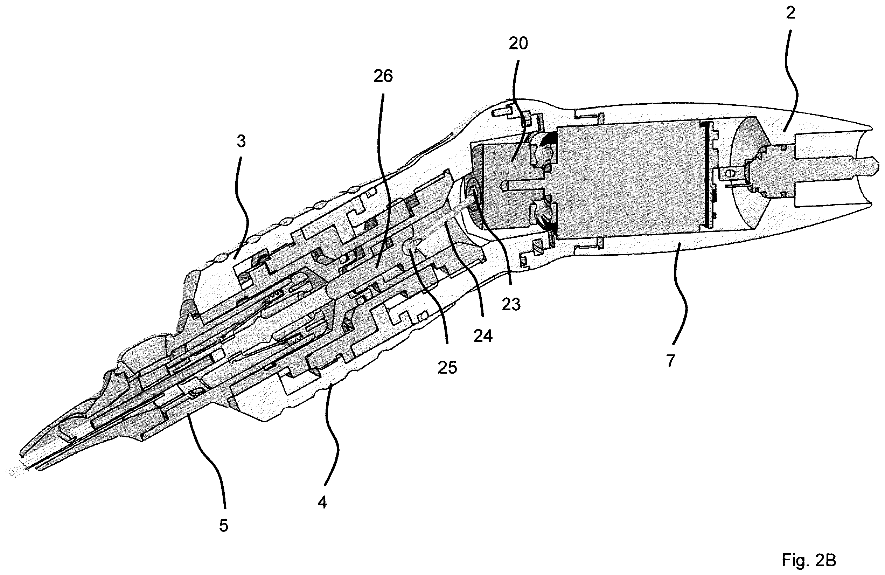

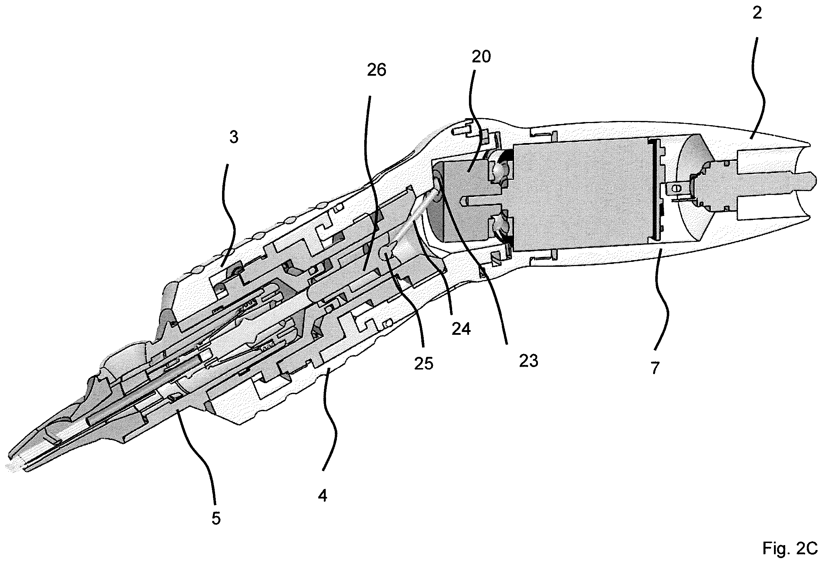

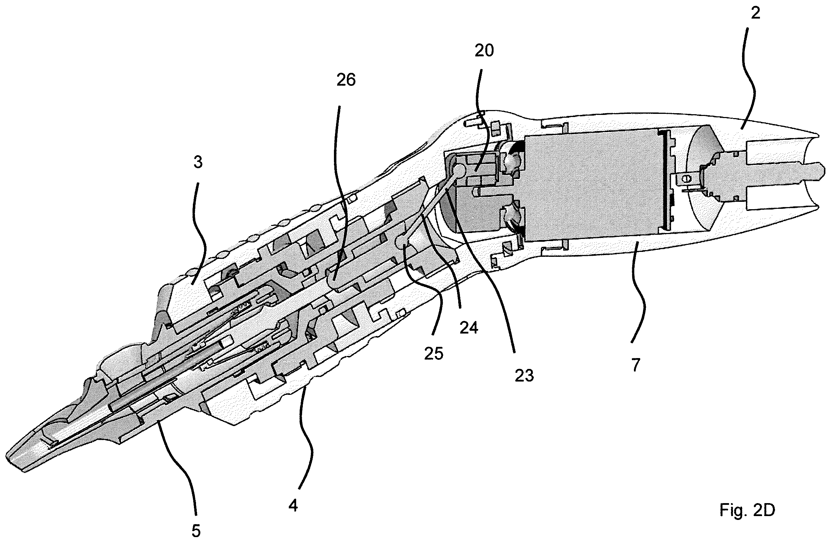

Fig. 2A bis 2D zeigen das Handgerät 1 im Schnitt in verschiedenen Arbeitsstellungen. Die verschiedenen Arbeitsstellungen unterscheiden sich durch verschiedene Drehstellungen eines antriebsseitigen Kopplungselementes 20, welches auf eine Antriebswelle 21 einer Antriebseinrichtung 22 sitzt. Bei der Antriebseinrichtung 22 handelt es sich beispielsweise um einen Elektromotor. Mit Hilfe der Antriebseinrichtung 22 wird die Antriebswelle 21 in eine Rotationsbewegung versetzt, so dass hierdurch das antriebsseitige Kopplungselement 20 gedreht wird. Das antriebsseitige Kopplungselement 20 steht gelenkig mit einem proximalen Pleuelende 23 eines Stabpleuels 24 in Verbindung. Bei der dargestellten Ausführungsform ist die gelenkige Verbindung mit Hilfe eines Kugelgelenkes hergestellt. Aufgrund der rotierenden Bewegung des antriebsseitigen Kopplungselementes 20 bewegt sich das antriebsseitige Pleuelende 23 auf einer geschlossenen Bewegungsbahn um die Antriebswelle 21 herum. Die Fig. 2A bis 2D zeigen verschiedene Drehstellungen des antriebsseitigen Kopplungselementes 20.

Ein distales Pleuelende 25 des Stabpleuels 24 ist an einem abtriebsseitigen Kopplungselement 26 gelenkig gelagert, im gezeigten Ausführungsbeispiel ebenfalls mithilfe eines Kugelgelenkes. Mithilfe des antriebsseitigen Kopplungselementes 20, des abtriebsseitigen Kopplungselementes 26 sowie des Stabpleuels 24 ist ein Wandlungsmechanismus bereitgestellt, mit dem die rotierende Antriebsbewegung der Antriebswelle 21 in eine axiale Vor- und Zurückbewegung des abtriebsseitigen Kopplungselementes 26 in einer Führung 27 umgewandelt wird. Aufgrund der rotierenden Bewegung des antriebsseitigen Kopplungselementes 20 wird über die Kopplung mit dem Stabpleuel 24 eine repetierende Vorwärts- und Rückwärtsbewegung des abtriebsseitigen Kopplungselementes 26 erzwungen. Die hierbei ausgeführte axiale Bewegung des abtriebsseitigen Kopplungselementes 26 weist eine Bewegungsrichtung auf, die mit der axialen Richtung der Antriebswelle 21 nicht parallel gebildet ist, sondern vielmehr in einem Winkel hierzu (Schrägstellung) steht.

An das abtriebsseitige Kopplungselement 26 koppelt ein Zwischenstück 28, an dessen Vorderseite in einer Aufnahme 29 ein Nadelschaft 30 einer Stecheinrichtung 31 aufgenommen ist. Die Stecheinrichtung 31 weist mehreren Nadeln 8 auf, die ihrerseits an dem Nadelschaft 30 aufgenommen sind. In einer anderen Ausführung kann auch nur eine Nadel vorgesehen sein. Im Betrieb bewegt sich eine Stechnadelspitze 32 vor und zurück in einer vorderen Gehäuseöffhung 9 des Nadelmoduls 5, an welchem eine Modulspitze 5a gebildet ist.

Es ist eine Rückstelleinrichtung 33 mit einer Membran 34 vorgesehen. Die Membran 34 wird in axialer Richtung gestreckt, wenn der Nadelschaft 30 mit den hierin aufgenommenen Nadeln 8 nach vorne bewegt wird, derart, dass die Membran 34 eine über eine Vorspannkraft hinausgehende Rückstellkraft bereitstellt. Diese wirkt ergänzend zu einer durch den Stabpleuel 24 erzwungenen Rückbewegung.

Bei der in den Fig. 2A bis 2D dargestellten Ausführungsform ist ein Arbeitshub des Nadelschaftes 30 beim Vor- und Zurückbewegen maximal eingestellt. Dies erfolgt mittels Verdrehen von vorderem und hinterem Gehäuseteil 3, 7 relativ zueinander, die hierzu über eine Gelenkverbindung 35, 36 miteinander verbunden sind. Aufgrund des Verdrehens ändert sich der Winkel der Schrägstellung zwischen Antriebsachse und Achse der Vor- und Zurückbewegung der Nadel 8, was den Abstand der Lagerungen für das antriebsseitige sowie das abtriebsseitige Pleuelende 23, 25 verändert. Die Kopplung von vorderem und hinterem Gehäuseteil 3, 7 erfolgt hierbei unter Beteiligung eines Kunststoffringes 36.

Ein Nadelüberstand 37 der Nadelspitze in Bezug zu der vorderen Gehäuseöffnung 9 ist ebenfalls maximal. Der Nadelüberstand 37 kann eingestellt werden. Hierzu ist ein Einstellbauteil 38 mittels einer Schraubverbindung 39 gelagert. Mittels Drehen (Schrauben) des Einstellbauteils 38, welches hierdurch seine Relativlage längs der Gehäuseachse im vorderen Gehäuseteil 3 ändert, erfolgt eine Einstellung des Nadelüberstandes 37.

Eine Abschlusskappe 40 sitzt rückseitig an dem Nadelmodel 5.

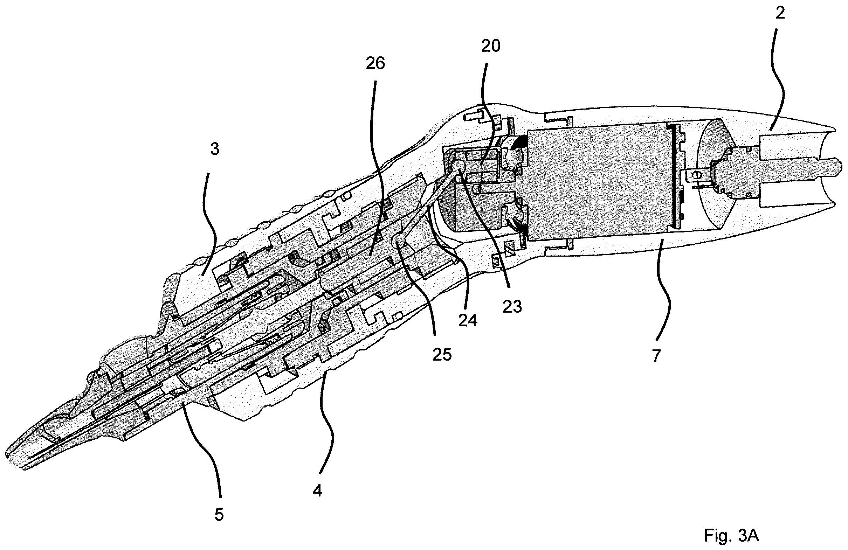

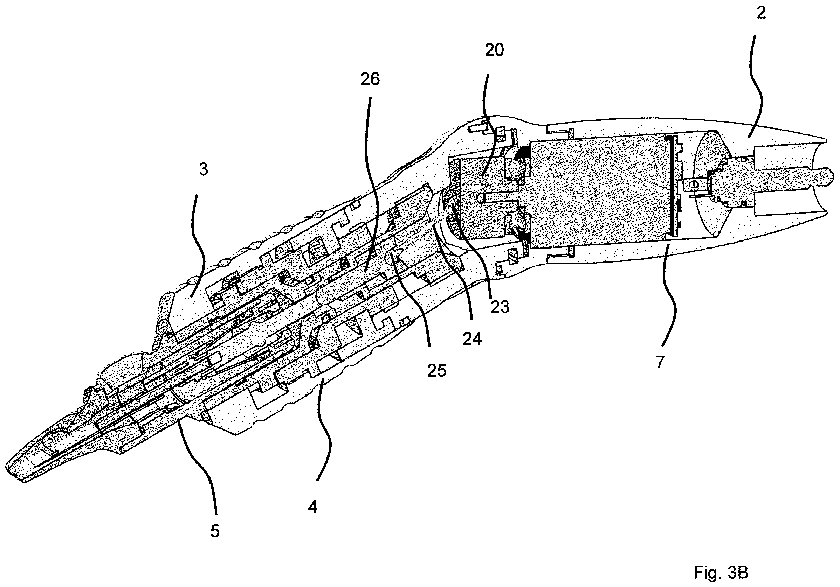

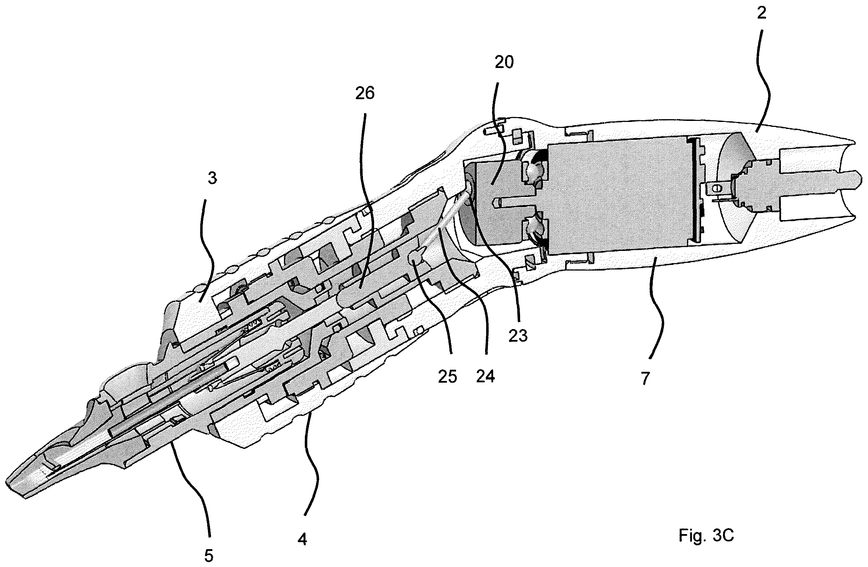

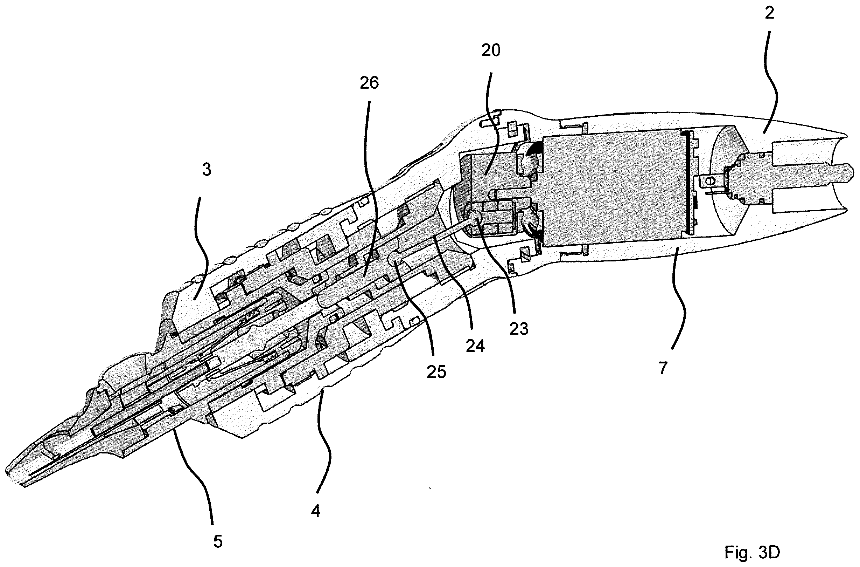

Die Fig. 3A bis 3D zeigen das Handgerät 1 vergleichbar zu den Fig. 2A bis 2D in verschiedenen Arbeitsstellungen, wobei bei dieser Ausgestaltung der Hubes des Nadelschaftes 30 ebenfalls maximal eingestellt ist. Im Unterschied zu den Fig. 2A bis 2D ist nun aber der Nadelüberstand minimal eingestellt.

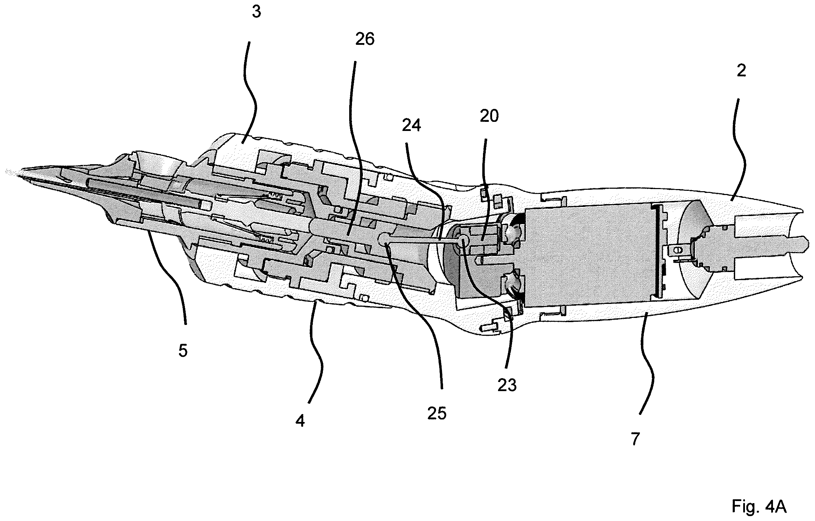

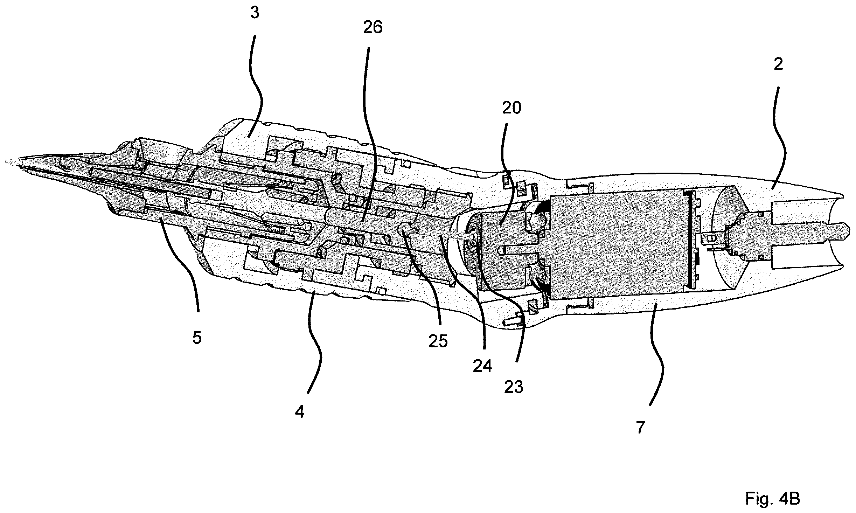

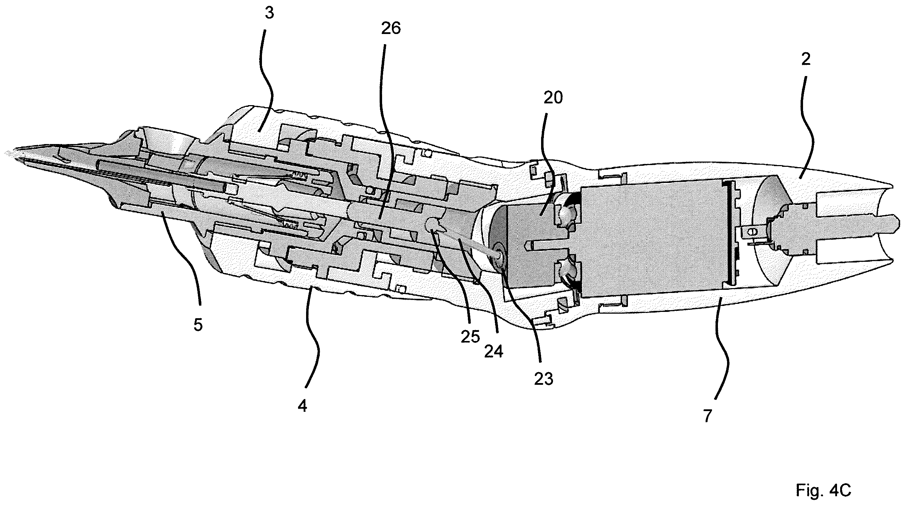

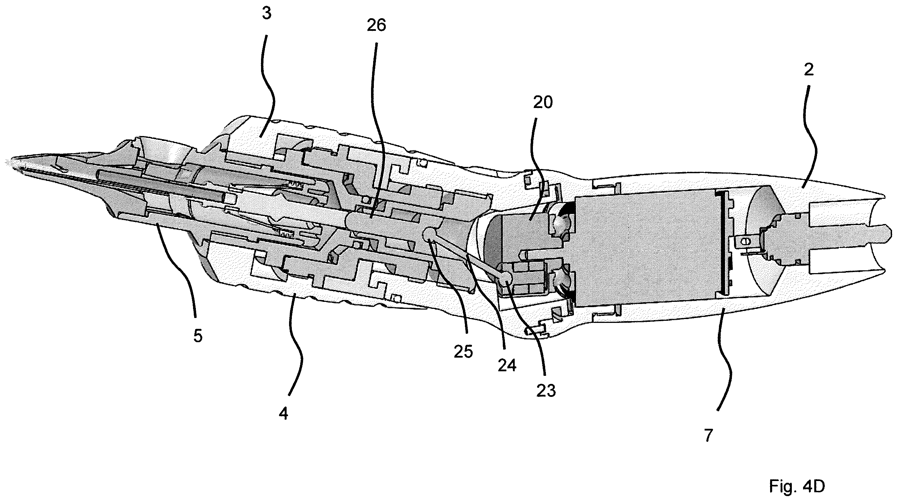

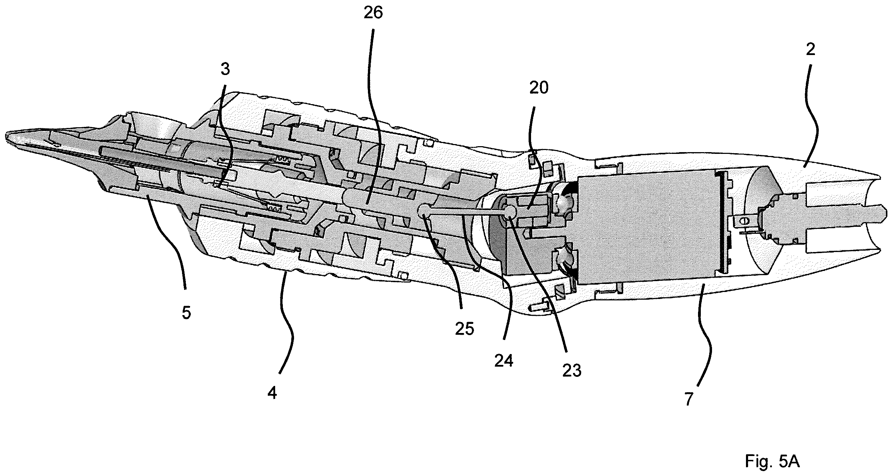

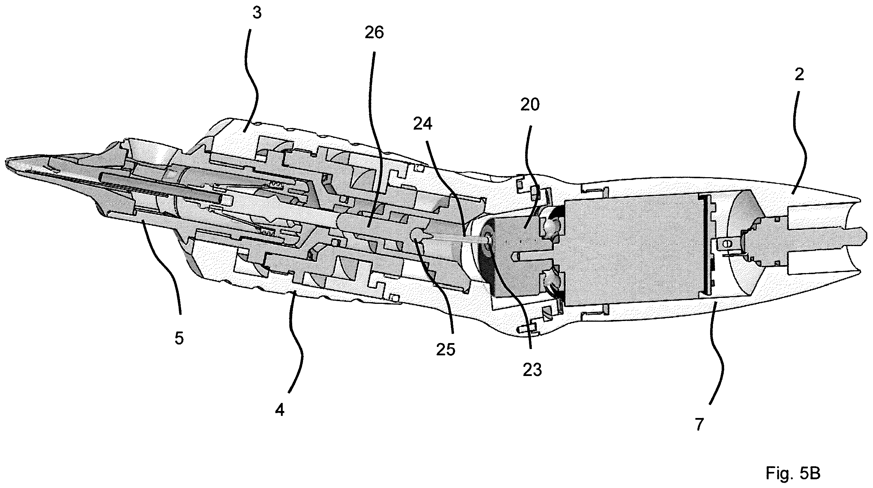

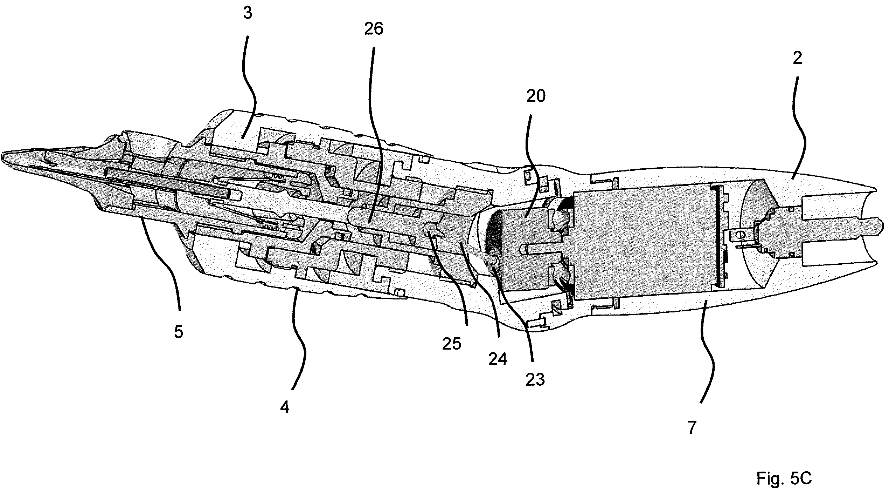

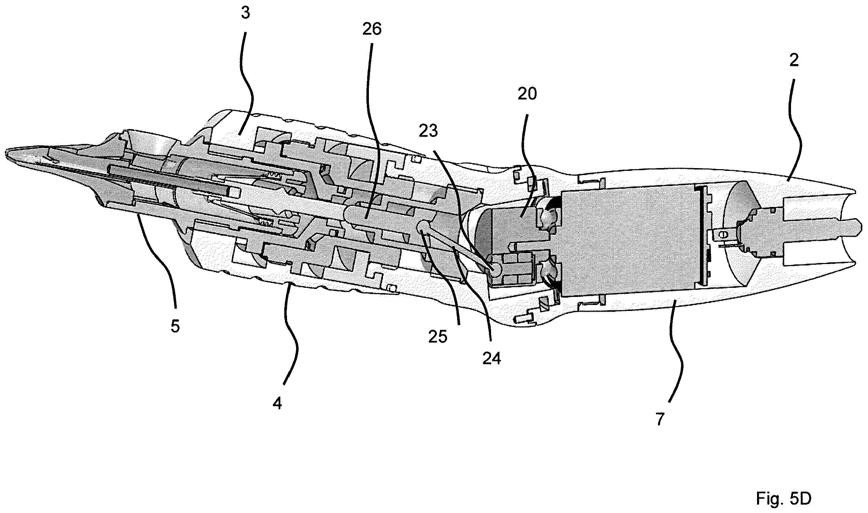

Die Fig. 4A bis 4D sowie 5A bis 5D zeigen das Handgerät 1 ebenfalls in verschiedenen Arbeitsstellungen, wobei der Hub des Nadelschaftes 30 nun minimiert ist. Bei der Ausführungsform in den Fig. 4A bis 4D ist der Nadelüberstand maximiert, wohingegen bei der Ausgestaltung in den Fig. 5A bis 5D der Nadelüberstand wieder minimiert ist.

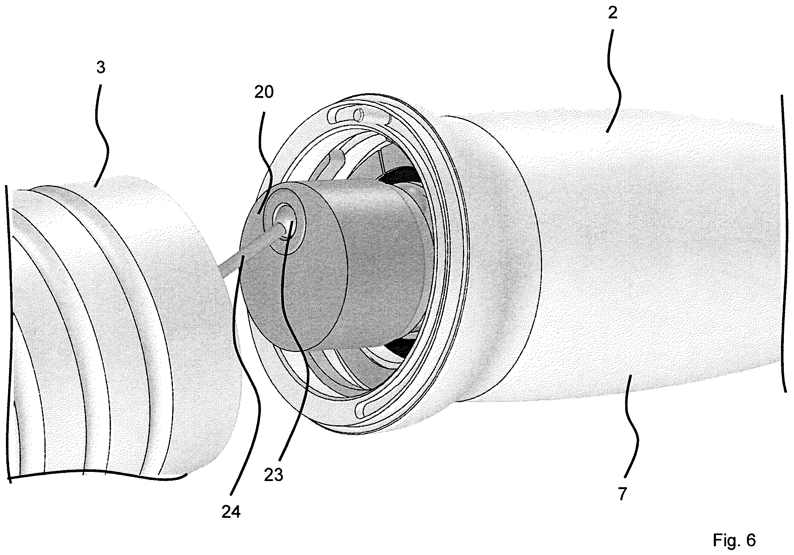

Fig. 6 zeigt eine vergrößerte perspektivische Darstellung eines Abschnitts des Handgeräts 1 mit Teilen des Gehäuses 2, wobei insbesondere das antriebsseitige Kopplungselement 20 sowie das hieran koppelnde antriebsseitige Pleuelende 23 gezeigt und das mit dem Gehäuse 2 um einen begrenzten Winkel drehbar koppelnde, vordere Gehäuseteil 3 von Gehäuse ausgeblendet sind.

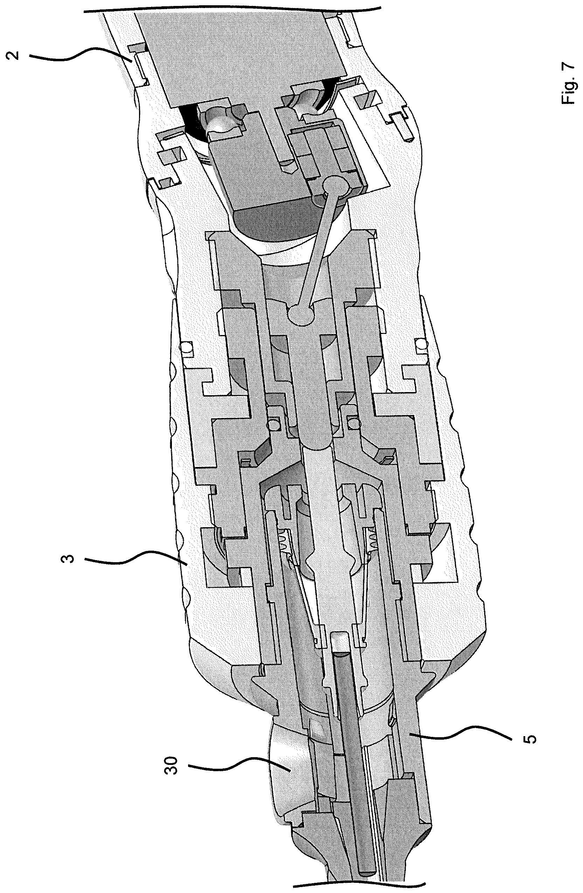

Fig. 7 zeigt eine vergrößerte Schnittdarstellung des Handgeräts 1.

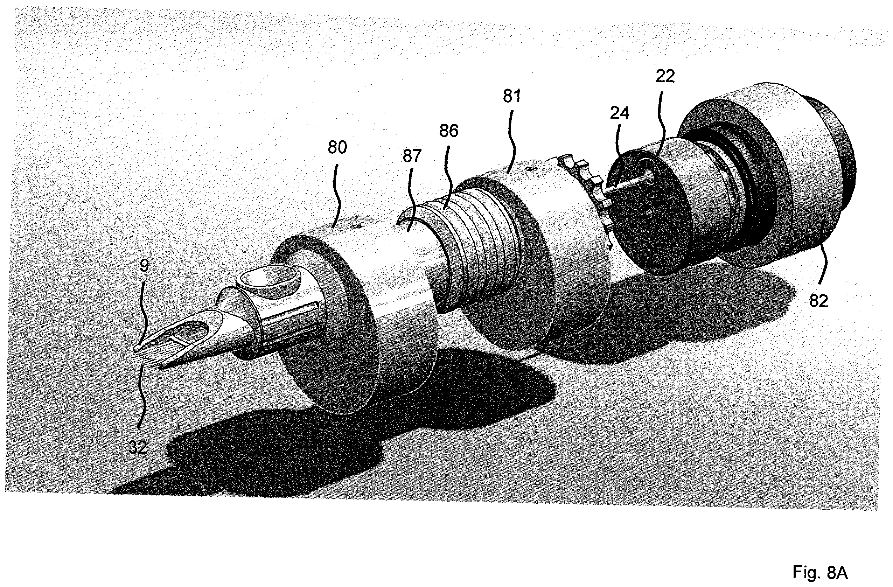

Fig. 8A zeigt eine schematische perspektivische Darstellung einer Anordnung für ein Handgerät zum wiederholten Aufstechen einer Haut, die nicht Teil der beanspruchten Erfindung ist, bei dem im Unterschied zu den Ausführungsformen in den Fig. 1 bis 7 die Achse der Antriebswelle 21 sowie der Verlauf der geradlinigen Bewegungsbahn beim Vor- und Zurückbewegen der Stecheinrichtung 31 parallel versetzt zueinander angeordnet sind. Fig. 8A zeigt hierbei einen experimentellen Aufbau, bei dem Komponenten des Handgerätes zu Untersuchungs- und Testzwecken an Adaptern 80, 81, 82 angeordnet sind. Für gleiche Merkmale werden hier im Übrigen dieselben Bezugszeichen wie bei der vorangehenden Beschreibung verwendet.

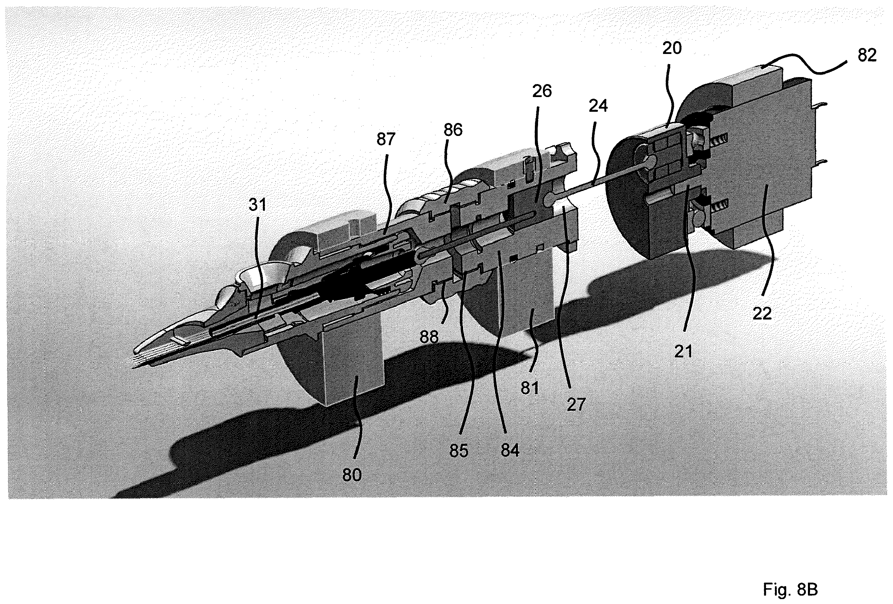

Die Fig. 8B bis 8D zeigen die Anordnung aus Fig. 8A in unterschiedlichen Betriebsstellungen für das antriebsseitige Kopplungselement 20 sowie verschiedene Einstellungen für einen von der Stecheinrichtung 31 ausgeführten Hub und einen maximalen Nadelherausstand.

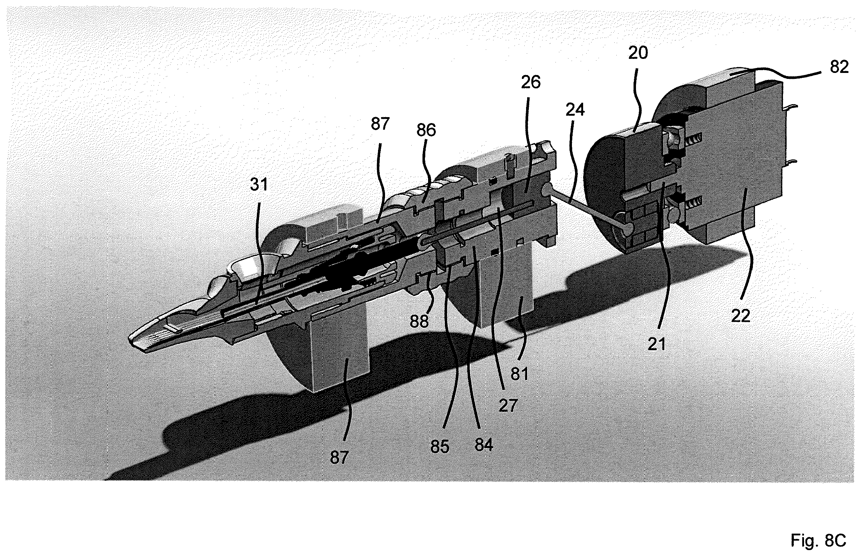

Bei der Ausgestaltung in den Fig. 8B und 8C ist mittels einer Hubeinstellung der von der Stecheinrichtung 31 ausgeführte Hub maximal eingestellt. In Fig. 8B ist der Abstand zwischen antriebsseitigem und abtriebsseitigem Kopplungselement 20, 26 maximal, in Fig. 8C minimal, wobei die Überführung des in Fig. 8B gezeigten Zustands in den in Fig. 8C gezeigten durch eine 180°-Drehung des antriebsseitigen Kopplungselements 20 erreicht wird.

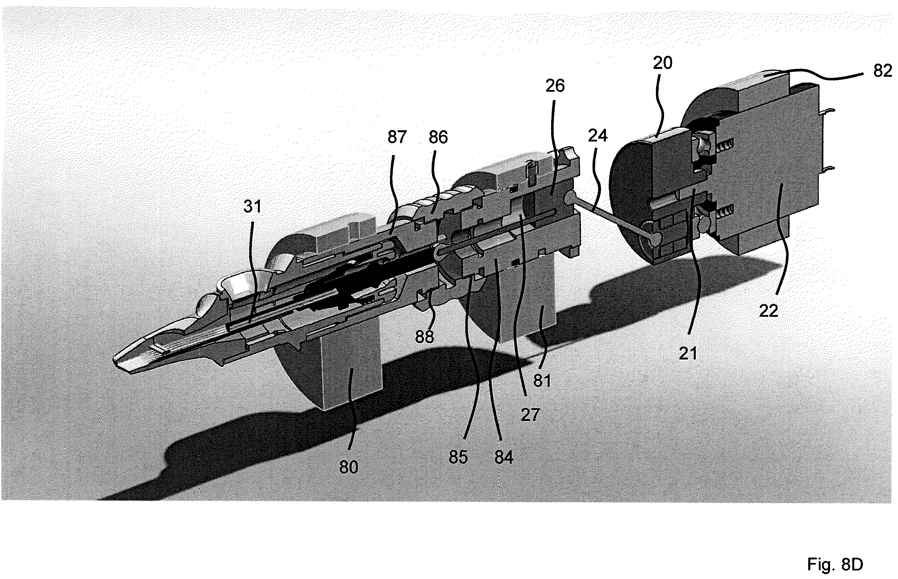

Bei der Darstellung in Fig. 8D ist der ausgeführte Hub minimal eingestellt, der Abstand zwischen antriebsseitigem und abtriebsseitigem Kopplungselement 20, 26 ist ebenfalls minimal. Die Hubeinstellung erfolgt bei dieser Gestaltungsvariante mittels Verdrehen eines in exzentrischer Lage zur Antriebslängsachse dreh- und verschiebbar angeordneten Bauteils 84, in welchem wiederum in exzentrischer Lage zu dessen Mittelachse die Führung 27 für das abtriebsseitige Kopplungselement 26 gebildet ist. Wird das Bauteil 84 um seine Längsachse gedreht, wird die Mittelachse des abtriebsseitigen Koppelelements 26 und damit die abtriebsseitige Gelenkpfanne, welche das abtriebsseitige Pleuelende 25 aufnimmt, der Antriebslängsachse (axiale Richtung der Antriebswelle 21) mittels additiver oder subtraktiver Überlagerung der Exzentrizitäten genähert oder davon entfernt. Hierdurch wird der Hub verkleinert oder vergrößert. Konstruktiv resultiert aus dieser Hubeinstellung insbesondere eine Verlagerung eines hinteren Totpunkts der Nadelbewegung der Stecheinrichtung 31. Hierbei wird die Hubeinstellung bewirkt mittels Ändern einer Relativlage zwischen der Bewegungsrichtung der Nadelbewegung der Stecheinrichtung 31 einerseits, insbesondere der Bewegungsrichtung der Bewegung des abtriebsseitigen Koppelelements 26 in der Führung 27, und der axialen Richtung der Antriebswelle 21. Es findet ein Parallelversatz statt. Der Abstand zwischen den beiden parallel verlaufenden Richtungen (Achsen) wird verändert, um den Hub einzustellen.

Weiterhin ist ein Nadelherausstand einstellbar, also der Umfang des Überstandes der Stechnadelspitze 32 in Relation zur vorderen Gehäuseöffnung 9. Bei der Ausgestaltung in Fig. 8B und 8C ist der Nadelheraus- oder Nadelüberstand maximal, wohingegen er bei der Ausgestaltung in Fig. 8D minimal ist. Die Einstellung des Nadelherausstands erfolgt zum Beispiel mittels Drehens einer Einstellhülse 86, in welcher eine Modulaufnahme 87 mittels einer Schraubverbindung 88 gelagert ist.

Um einen Nadelherausstand bei der Hubeinstellung festzuhalten (konstant zu halten), erfolgt bei der gezeigten Ausführung mit der Drehung des Bauteils 84 zur Hubeinstellung dank einer Steuerkurve eine drehwinkelabhängige axiale Verlagerung aller weiter distal angeordneten Bauteile. Dazu greift beispielsweise ein Gewindestift in eine auf dem Umfang der Einstellhülse 86 angebrachte Steuerkurve, die wiederum durch ihre Drehwinkel-Steigungs-Funktion eine fest vorgegebene axiale Verschiebung um einen definierten Betrag je Drehwinkeländerung bewirkt. Die Hubeinstellung ohne die beschriebene Kompensation kann als kombinierte Nadelherausstands-Hub-Einstellung aufgefasst werden, so dass auf eine separate Einstellung des Nadelherausstands verzichtet werden kann.

Die Drehbewegung zur Hubeinstellung kann von der Winkelstellung des Stechmoduls entkoppelt sein.

Bei einer auf Fig. 8 aufbauende Gestaltungsvariante (nicht dargestellt und auch nicht Teil der beanspruchten Erfindung), kann ein Ausgleichsmechanismus vorgesehen sein, welcher geeignet ist, den Achsenversatz zwischen der Antriebsachse und der Längsachse des abtriebsseitigen Koppelelements 26 derart auszugleichen, dass das Stechmodul 5 in der antriebsseitigen Achse zu liegen kommt und die Stechbewegung weitestgehend in dieser Achse erfolgt.

Eine Achsenverlagerung zur Hubeinstellung kann statt vermittels einer Drehbewegung auch durch eine Verschiebung oder durch deren Kombination oder Ausführung hintereinander erfolgen.

Der Wandlungsmechanismus, welcher insbesondere das antriebsseitige Kopplungselement 22, das Stabpleuel 24 sowie das abtriebsseitige Kopplungselement 26 umfasst, kann bei den verschiedenen Ausgestaltungen des Handgerätes ganz oder teilwiese im Stechmodul 5 ausgebildet sein, wobei eine lösbare Drehmomentübertragung der Antriebseinrichtung 22 auf den Wandlungsmechanismus vorgesehen sein kann, also in Fig. 8B zwischen dem antriebsseitigen Kopplungselement 20 und der Antriebswelle 21 der Antriebseinrichtung 22.

Die Stechbewegung kann für alle Varianten dieses Antriebskonzepts auch in die entgegengesetzte Achsenrichtung erfolgen, beispielsweise wenn der vordere Totpunkt durch die kombinierte Hub-Nadelherausstand-Einstellung beeinflusst werden soll oder es der Bauraum erforderlich macht, beispielsweise, wenn ein kompaktes kurzes Stechgerät gewünscht wird statt eines langgestreckten stiftförmigen.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Handgerät zum wiederholten lokalen Aufstechen einer menschlichen oder einer tierischen Haut, mit:
- einem Gehäuse (2), an dem ein Handgriff (4) gebildet ist,
- einer Antriebseinrichtung (22), die in dem Gehäuse (2) angeordnet ist und mit der über eine Antriebswelle (21) eine rotierende Bewegung um eine Drehachse bereitgestellt wird,
- einem an die Antriebswelle (21) koppelnden Wandlungsmechanismus (20, 24, 26), der in dem Gehäuse (2) angeordnet und eingerichtet ist, die rotierende Bewegung in eine Antriebsbewegung entlang einer Antriebsbewegungsrichtung für eine Vor- und Zurückbewegung zu wandeln, und
- einer Stecheinrichtung (31), die in dem Gehäuse (2) angeordnet ist und eine Stechnadel (8) aufweist, welche an einem Nadelschaft (30) angeordnet ist, der zusammen mit der Stechnadel (8) entlang einer Bewegungsbahn wiederholt vor und zurück bewegbar ist und mit dem Wandlungsmechanismus (20, 24, 26) in Verbindung steht,
wobei der Wandlungsmechanismus (20, 24, 26) eine Stabkurbeleinrichtung aufweist und ein von der Stechnadel (8) beim Vor- und Zurückbewegen ausgeführter Hub einstellbar ist, indem eine Relativlage zwischen einer Axialrichtung der Drehachse und der Antriebsbewegungsrichtung verändert wird,
**dadurch gekennzeichnet, dass** die Antriebsbewegungsrichtung nicht parallel zur Axialrichtung der Drehachse angeordnet ist.

2. Handgerät nach Anspruch 1, **dadurch gekennzeichnet, dass** der bei Stabkurbeleinrichtung
- ein an die Antriebswelle (21) koppelndes antriebseitiges Kopplungselement (20), welches im Betrieb der rotierenden Bewegung entsprechend rotiert,
- ein proximales Pleuelende (23) eines Stabpleuels (24) an das antriebseitige Kopplungselement (20) gelenkig koppelt, derart, dass das proximale Pleuelende (23) beim Rotieren des antriebseitigen Kopplungselementes (20) auf einer geschlossenen Bewegungsbahn um die Rotationssachse der Antriebswelle (21) herum bewegt wird, und
- ein distales Pleuelende (25) des Stabpleuels (24) an ein abtriebseitiges Kopplungselement (26) gelenkig koppelt, welches zum Übertragen der Antriebsbewegung an den Nadelschaft (30) koppelt.

3. Handgerät nach Anspruch 1 oder 2, **dadurch gekennzeichnet, dass** der Wandlungsmechanismus (20, 24, 26) eine Drehentkopplung aufweist.

4. Handgerät nach Anspruch 3, **dadurch gekennzeichnet, dass** die gelenkige Kopplung zwischen dem proximalen Pleuelende (23) und dem antriebseitigen Kopplungselement (20) drehentkoppelnd ausgeführt ist.

5. Handgerät nach Anspruch 3 oder 4, **dadurch gekennzeichnet, dass** die gelenkige Kopplung zwischen dem distalen Pleuelende (25) und dem abtriebseitigen Kopplungselement (26) drehentkoppelnd ausgeführt ist.

6. Handgerät nach mindestens einem der vorangehenden Ansprüche, soweit auf Anspruch 2 rückbezogen, **dadurch gekennzeichnet, dass** das proximale Pleuelende (23) und das distale Pleuelende (25) am Stabpleuel (24) drehentkoppelt aufgenommen sind.

7. Handgerät nach mindestens einem der vorangehenden Ansprüche, soweit auf Anspruch 2 rückbezogen, **dadurch gekennzeichnet, dass** das proximale Pleuelende (23) mit dem antriebseitigen Kopplungselement (20) und / oder das distale Pleuelende (25) mit dem abtriebseitigen Kopplungselement (26) mittels eines Kugelgelenkes gelenkig verbunden sind.

8. Handgerät nach mindestens einem der vorangehenden Ansprüche, soweit auf Anspruch 2 rückbezogen, **dadurch gekennzeichnet, dass** das abtriebseitige Kopplungselement (26) über eine starre Verbindung mit dem Nadelschaft (30) verbunden oder einstückig hiermit ausgeführt ist.

9. Handgerät nach mindestens einem der vorangehenden Ansprüche, **dadurch gekennzeichnet, dass** an den Nadelschaft (30) eine Rückholeinrichtung (34) koppelt.

10. Handgerät nach mindestens einem der vorangehenden Ansprüche, **dadurch gekennzeichnet, dass** mittels des Einstellens des Hubs ein maximaler Nadelüberstand (37) einer Stechnadelspitze (32) der Stechnadel (8) in Bezug auf eine vordere Gehäuseöffnung (9) einstellbar ist.

11. Handgerät nach mindestens einem der vorangehenden Ansprüche, **dadurch gekennzeichnet, dass** das Gehäuse (2) mit mehreren Gehäusemodulen (3, 7) gebildet ist, wobei in einem Antriebsmodul die Antriebseinrichtung (22) und in einem Stech- oder Nadelmodul (5) die Stecheinrichtung (31) angeordnet sind.

## Claims

1. A handheld device for repeated local puncture of human or animal skin, comprising:
- a housing (2) on which a handle (4) is farmed.
- a drive device (22) which is disposed in the housing (2) and with which a rotational movement about an axis of rotation is provided via a driveshaft (21),
- a conversion mechanism (20, 24, 26) which is coupled to the driveshaft (21) and is disposed in the housing (2) and configured to convert the rotational movement into a drive movement along a drive movement direction to produce a back-and-forth movement, and
- a puncture device (31), which is disposed in the housing (2) and which comprises a puncture needle (8) which is disposed on a needle shaft (30) and which can be repeatedly moved back and forth together with the puncture needle (8) along a path of movement and which is connected to the conversion mechanism (20, 24, 26),
wherein the conversion mechanism (20, 24, 26) comprises a rod crank device, and a stroke executed by the puncture needle (8) when moving back and forth is adjustable by altering a relative position between an axial direction of the axis of rotation and the direction of the drive movement,
**characterized in that** the direction of the drive movement is not parallel to the axial direction of the axis of rotation.

2. The handheld device according to claim 1, **characterized in that** in the rod crank device
- a drive-side coupling element (20) is provided which is coupled to the driveshaft (21) and rotates in a manner corresponding to the rotational movement during operation,
- a proximal connecting rod end (23) of a connecting rod (24) is coupled to the drive-side coupling element (20) in an articulated manner, such that the proximal connecting rod end (23) is moved around a closed path of movement about the axis of rotation of the drive shaft (21) during rotation of the drive-side coupling element (20), and
- a distal connecting rod end (25) of the connecting rod (24) is coupled to an output-side coupling element (26) in an articulated manner which is coupled to the needle shaft (30) in order to transfer the drive movement.

3. The handheld device as claimed in claim 1 or claim 2, **characterized in that** the conversion mechanism (20, 24, 26) comprises a rotational decoupling.

4. The handheld device as claimed in claim 3, **characterized in that** the articulated coupling between the proximal connecting rod end (23) and the drive-side coupling element (20) is configured so as to be rotationally decoupling.

5. The handheld device as claimed in claim 3 or claim 4, **characterized in that** the articulated coupling between the distal connecting rod end (25) and the output-side coupling element (26) is configured so as to be rotationally decoupling.

6. The handheld device as claimed in at least one of the preceding claims insofar as it is dependent on claim 2, **characterized in that** the proximal connecting rod end (23) and the distal connecting rod end (25) are accommodated on the connecting rod (24) in a rotationally decoupled manner.

7. The handheld device as claimed in at least one of the preceding claims insofar as it is dependent on claim 2, **characterized in that** the proximal end of the connecting rod (23) is connected to the drive-side coupling element (20) and/or the distal connecting rod end (25) is connected to the output-side coupling element (26) in an articulated manner by means of a ball joint.

8. The handheld device as claimed in at least one of the preceding claims insofar as it is dependent on claim 2, **characterized in that** the output-side coupling element (26) is connected to the needle shaft (30) via a rigid connection or is formed in one piece therewith.

9. The handheld device as claimed in at least one of the preceding claims, **characterized in that** a restoring device (34) is coupled to the needle shaft (30).

10. The handheld device as claimed in at least one of the preceding claims, **characterized in that** a maximum needle protrusion (37) of a puncture needle tip (32) of the puncture needle (8) is adjustable with respect to a front housing opening (9) by means of the adjustment to the stroke.

11. The handheld device as claimed in at least one of the preceding claims, **characterized in that** the housing (2) is formed with a plurality of housing modules (3, 7), wherein the drive device (22) is disposed in a drive module and the puncture device (31) is disposed in a puncture module or needle module (5).

## Revendications

1. Appareil manuel permettant de piquer localement à répétition une peau humaine ou animale, comportant :
- un boîtier (2) sur lequel une poignée (4) est constituée,
- un dispositif d'entraînement (22) qui est disposé dans le boîtier (2) et grâce auquel un mouvement de rotation autour d'un axe de rotation est fourni par un arbre moteur (21),
- un mécanisme de conversion (20, 24, 26) se couplant à l'arbre moteur (21) et qui est disposé dans le boîtier (2) et conçu pour transformer le mouvement de rotation en un mouvement d'entraînement le long d'un sens de mouvement d'entraînement pour un mouvement aller et retour, et
- un dispositif de piquage (31) qui est disposé dans le boîtier (2) et présente une aiguille de piquage (8) qui est disposée sur une tige d'aiguille (30) qui est mobile en aller-retour à répétition avec l'aiguille de piquage (8) le long d'une piste de mouvement et qui est en liaison avec le mécanisme de conversion (20, 24, 26),
le mécanisme de conversion (20, 24, 26) présentant un dispositif de manivelle de bielle et une course exécutée par l'aiguille de piquage (8) lors du mouvement aller-retour étant réglable par le fait qu'une position relative entre un sens axial de l'axe de rotation et le sens de mouvement d'entraînement est modifiée,
**caractérisé en ce que** le sens de mouvement d'entraînement est disposé non parallèlement au sens axial de l'axe de rotation.

2. Appareil manuel selon la revendication 1, **caractérisé en ce qu'**an dispositif de manivelle de bielle,
- un élément de couplage situé du côte moteur (20) se couple à l'arbre moteur (21) et qui tourne en conséquence pendant l'exécution du mouvement rotatif,
- une extrémité proximale de bielle en forme de barre (23) d'une bielle en forme de barre (24) couple de manière articulée à l'élément de couplage situé du côté moteur (20) de manière à ce que l'extrémité proximale de bielle (23), lors de la rotation de l'élément de couplage situé du côté moteur (20) sur une piste de mouvement fermée, se déplace autour de l'axe de rotation de l'arbre moteur (21), et
- une extrémité distale de bielle (25) de la bielle en forme de barre (24) couple de manière articulée un élément de couplage (26) situé du côté mené et qui se couple à la tige d'aiguille (30) pour transférer le mouvement d'entraînement.

3. Appareil manuel selon la revendication 1 ou 2, **caractérisé en ce que** le mécanisme de conversion (20, 24, 26) présente un système de désaccouplage de rotation.

4. Appareil manuel selon la revendication 3, **caractérisé en ce que** le couplage articulé entre l'extrémité proximale de bielle (23) et l'élément de couplage situé du côté moteur (20) est réalisé de manière à désaccoupler la rotation.

5. Appareil manuel selon la revendication 3 ou 4, **caractérisé en ce que** le couplage articulé entre l'extrémité distale de bielle (25) et l'élément de couplage situé du côté mené (26) est réalisé de manière à désaccoupler la rotation.

6. Appareil manuel selon au moins une des revendications précédentes, dans la mesure où elles se réfèrent à la revendication 2, **caractérisé en ce que** l'extrémité proximale de bielle (23) et l'extrémité distale de bielle (25) sont reçues désaccouplées en rotation au niveau de la bielle en forme de barre (24).

7. Appareil manuel selon au moins une des revendications précédentes, dans la mesure où elles se réfèrent à la revendication 2, **caractérisé en ce que** l'extrémité proximale de bielle (23) est raccordée de manière articulée à l'élément de couplage situé du côté moteur (20) et/ou l'extrémité distale de bielle (25) à l'élément de couplage situé du côté mené (26) au moyen d'un joint à rotule.

8. Appareil manuel selon au moins une des revendications précédentes, dans la mesure où elles se réfèrent à la revendication 2, **caractérisé en ce que** l'élément de couplage situé du côté mené (26) est raccordé par une connexion rigide à la tige d'aiguille (30) ou est réalisé de manière à former une seule pièce avec celui-ci.

9. Appareil manuel selon au moins une des revendications précédentes, **caractérisé en ce qu'**un dispositif de récupération (34) se couple à la tige d'aiguille (30).

10. Appareil manuel selon au moins une des revendications précédentes, **caractérisé en ce que**, au moyen d'un réglage de la course, un surplomb maximal d'aiguille (37) d'une pointe d'aiguille de piquage (32) de l'aiguille de piquage (8) est réglable par rapport à une ouverture avant du boîtier (9).

11. Appareil manuel selon au moins une des revendications précédentes, **caractérisé en ce que** le boîtier (2) est réalisé avec plusieurs modules de boîtier (3, 7), le dispositif moteur (22) étant disposé dans un module d'entraînement et le dispositif de piquage (31) dans un module de piquage ou d'aiguille (5).
